(19) **Europäisches Patentamt**
**European Patent Office**
**Office européen des brevets**

(11) **EP 1 923 027 B1**

(12) **EUROPÄISCHE PATENTSCHRIFT**

(45) Veröffentlichungstag und Bekanntmachung des Hinweises auf die Patenterteilung:
**11.01.2012 Patentblatt 2012/02**

(51) Int Cl.:
*A61F 9/01* *(2006.01)* *A61B 3/113* *(2006.01)*

(21) Anmeldenummer: **06023259.2**

(22) Anmeldetag: **08.11.2006**

(54) **System zum Ablatieren von Hornhaut an einem Auge**

System for controlling a corneal laser ablation of an eye

Système de contrôle de l'ablation de la cornée d'un oeil au moyen d'un laser

(84) Benannte Vertragsstaaten:
**DE ES FR IT**

(43) Veröffentlichungstag der Anmeldung:
**21.05.2008 Patentblatt 2008/21**

(73) Patentinhaber: **Schwind eye-tech-solutions GmbH & Co. KG**
**63801 Kleinostheim (DE)**

(72) Erfinder:
• **Arba-Mosquera, Samuel**
**63739 Aschaffenburg (DE)**

• **Magnago, Thomas**
**63762 Großostheim-Wenigumstadt (DE)**

(74) Vertreter: **Hofstetter, Alfons J. et al**
**Hofstetter, Schurack & Skora**
**Balanstrasse 57**
**81541 München (DE)**

(56) Entgegenhaltungen:
WO-A1-2005/099639    US-A1- 2003 223 037
US-A1- 2004 044 333    US-A1- 2005 119 642

**Beschreibung**

Die Erfindung betrifft ein System zum Ablatieren von Hornhaut an einem Auge nach dem Oberbegriff von Patentanspruch 1. Ein solches System ist aus der WO 2005/099639 A1 bekannt.

Zur Laserablation an einem Auge wird ein Laser verwendet, der pulsweise betrieben werden kann. Eine Zieleinrichtung lenkt die jeweiligen Laserpulse jeweils auf eine bestimmte Stelle der Augenhornhaut. Die Auftreffstelle auf der Augenhornhaut wird zu jedem Laserpuls einzeln festgelegt. Das sich insgesamt ergebende Muster dient dazu, genau eine bestimmte Kontur auf der Hornhaut abzutragen, damit bestimmte Linsenwirkungen der Hornhaut erzielt werden. Die jeweilige Auftreffstelle des Lasers auf der Augenhornhaut wird relativ zu einem Bezugsort auf der Augenhornhaut festgelegt. Da sich während der Laserbehandlung das Auge bewegt, müssen die Einstellungen der Zieleinrichtung in Abhängigkeit von Messwerten über die Augenstellung ständig angepasst werden. Hierbei wird ein so genannter Eyetracker verwendet. Der Eyetracker erkennt insbesondere das Zentrum der Pupille des Auges und erfasst zwei Koordinaten der Position dieses Zentrums. Diese beiden Koordinaten werden zur Definition eines Offsets der Zieleinrichtung verwendet. Der Eyetracker führt entsprechende Eingabedaten der Zieleinrichtung zur Herstellung einer Grundstellung zu. In der Grundstellung gleicht die Zieleinrichtung genau den Offset aus, der durch die Bewegung des Auges entstanden ist.

Bei einer solchen Offsetkorrektur bleibt der Unterschied unbeachtet, dass der Eyetracker das Zentrum der Pupille des Auges verfolgt, die im Auge etwas tiefer liegt, während der Laserstrahl auf die Hornhaut des Auges auftreffen soll, also auf die Oberfläche. So kommt es, dass bei einer Augenverkippung ein zu kleiner Offset erfasst wird.

Die WO 2005/099639 beschreibt ein Lasersystem für die Korrektur von Augenfehlern, bei dem bei einer Ablation von Hornhaut an einem Auge dessen Bewegungen überprüft werden. Es wird zwischen Translationsbewegungen und Kippbewegungen unterschieden. Für die Translationsbewegung wird ein gesonderter Eyetracker ("translation tracker") eingesetzt. Dieser ermittelt, um wieweit sich Punkte auf der Hornhaut geradlinig verschieben. Dieses Ergebnis wird mit Messungen eines weiteren Eyetrackers verglichen, um so auf die Verkippung des Auges zurückzuschließen. Man erhält so einen Korrekturvektor.

Es ist Aufgabe der Erfindung, ein System nach dem Oberbegriff von Patentanspruch 1 dahingehend weiterzubilden, dass seine Betriebsweise präziser wird.

Die Aufgabe wird durch ein System mit den Merkmalen von Patentanspruch 1 gelöst.

Es wird durch das System ein neuartiges Verfahren durchführbar. Dieses Verfahren umfasst somit die wiederholt durchlaufenden Schritte:

a) Erfassen von zwei Offsetkoordinaten der Position des Zentrums der Pupille des zu behandelnden Auges mit Hilfe eines Eyetrackers,
b) Korrigieren der Offsetkoordinaten,
c) Zielen des Lasers auf jeweils eine vordefinierte Stelle des Auges mit Hilfe einer Zieleinrichtung, wobei die Zieleinrichtung beim Zielen die korrigierten Offsetkoordinaten berücksichtigt, und Abgeben zumindest eines Laserschusses.

Es werden somit nicht mehr direkt die Koordinaten der Pupille für die Offsetkorrektur bei der Zieleinrichtung verwendet, sondern diese Offsetkoordinaten werden zuvor einer Korrektur unterworfen. Das Berücksichtigen der korrigierten Offsetkoordinaten erfolgt insbesondere so, dass die Zieleinrichtung in eine Grundstellung verbracht wird, welche genau den Offset, wie er durch die korrigierten Offsetkoordinaten quantifiziert ist, ausgleicht. Soll die Hornhaut des Auges zentral getroffen werden, so ist genau diese Grundstellung dieser Zieleinrichtung beim Zielen die richtige Einstellung. Ist die vordefinierte Stelle des Auges nicht zentral, so kann die Zieleinrichtung ausgehend von der durch die korrigierten Offsetkoordinaten definierten Grundstellung entsprechend eingestellt (verstellt) werden, um genau auf die vordefinierte Stelle zu zielen.

Bei der obigen Formulierung des Verfahrens ist somit davon ausgegangen, dass die Zieleinrichtung direkt mit dem Eyetracker verbunden ist und durch Eingabedaten von dem Eyetracker direkt in die Grundstellung verbracht wird. Während im Stand der Technik somit die von dem Eyetracker gemessenen Koordinaten direkt an die Zieleinrichtung weitergereicht werden, werden diese erfindungsgemäß aufbereitet.

Die Aufbereitung der Daten muss keineswegs aufwendig sein. Vielmehr ergeben einfache Überlegungen anhand des Strahlensatzes, dass die Offsetkoordinaten in Schritt b) des Verfahrens unter Umständen nur um konstante Faktor en $R_1$, $K_2$ korrigiert werden müssen.

Zudem Sind die Faktoren $k_1$, $k_2$ noch durch Messungen ermittelbar. Vorab an dem Auge wird gemessen:

-  der Abstand ZP von dem Zentrum der Pupille zur Oberfläche der Hornhaut an deren höchster Erhebung (also an der corneal vertex genannten Stelle, zu der dieser Abstand ZP im Übrigen am kleinsten ist),
-  der Abstand ZCoR von dem Mittelpunkt des Glaskörpers des Auges zur Oberfläche der Hornhaut an deren höchster Erhebung (an der Stelle, zu der dieser Abstand ZCoR am größten ist, also abermals am corneal vertex). Der konstante Faktor k ergibt sich dann nach dem Strahlensatz zu:

$$k = \frac{ZCoR}{ZCoR - ZP}.$$

Die genannten Vorabmessungen sind insbesondere dann vorteilhaft, wenn man eine besonders gute individuelle Anpassung des neuartigen Verfahrens an das Auge des Patienten wünscht.

Ein typischer Wert für ZP ist 3,75 mm, und ein typischer Wert für ZCoR ist 13 mm. Man kann auch diese typischen Werte verwenden und erhält so für den konstanten Faktor: k = 1,4. Damit lässt sich für k ein Bereich von 1,35 bis 1,45 definieren.

Einer möglichen Kritik, bei einer Korrektur mit einem Faktor von k = 1,4 würde davon ausgegangen werden, dass der Offset ausschließlich auf eine Verkippung des Auges zurückzuführen ist und nicht auf eine Bewegung des gesamten Kopfes mit dem Auge, lässt sich dadurch begegnen, dass von einem ca. hälftigen Anteil der Verkippung auf den Offset ausgegangen wird und als konstanter Faktor $1,17 \leq k \leq 1,23$ verwendet wird.

Eine Verfeinerung der bisher beschriebenen Vorgehensweise berücksichtigt, dass möglicherweise beim ersten Durchlauf vor dem ersten Laserschuss das Auge nicht vollständig koaxial zu dem Beobachtungssystem mit dem Eyetracker steht. Ein erster Offset kann somit nicht vollständig auf eine Verkippung des Auges zurückgeführt werden, nachfolgende Offsets aber schon. Dies lässt sich wie folgt rechnerisch ausdrücken: Beim ersten Durchlauf vor dem ersten Laserschuss werden die von dem Eyetracker ermittelten Offsetkoordinaten $X_{FS, ET}$, $y_{FS, ET}$, wobei "FS" für "First Shot", erster Laserschuss, steht und "ET" für "Eyetracker", um einen ersten Faktor $k_1$ korrigiert zu $X_{FS, korr} = k1 \, X_{FS, ET}$, $y_{FS, korr} = k_1 \, y_{FS, ET}$. Bei weiteren Durchläufen werden dann die Offsetkoordinaten $X_{LS, ET}$, $y_{LS, ET}$, wobei "LS" für "Later Shot", späterer Laserschuss, steht, korrigiert gemäß $X_{LS, korr} = X_{FS, korr} + k_2 (X_{LS, ET} - X_{FS, ET}) = k_2 \, X_{LS, ET} - (k_2 - k_1) \cdot X_{FS, ET}$ und analog für $y_{LS, korr} = k_2 \cdot y_{LS, ET} - (k_2 - k_1) \, y_{FS, ET}$, mit konstantem Faktor $k_2$.

Wie erwähnt, soll $k_1$ berücksichtigen, dass ein Anteil des Offsets auf eine Verkippung des Auges zurückzuführen ist. $k_1$ orientiert sich daher an einen Wert von 1,2, insbesondere kann man definieren: $1,0 \leq k_1 \leq 1,2$. $k_2$ hingegen soll berücksichtigen, dass die weitere Korrektur davon ausgeht, dass der zusätzliche Offset ausschließlich auf eine Verkippung zurückzuführen ist, so dass sich $k_2$ an dem oben genannten Wert von 1,4 orientiert. Es lässt sich definieren $1,2 \leq k_2 \leq 1,5$. $k_2$ ist hierbei größer als oder zumindest gleich k1.

Wie oben bereits erwähnt, geht die Erfindung davon aus, dass Koordinaten, wie sie ermittelt werden, zur Definition von Stellungen der Zieleinrichtung festgelegt werden. So ist es möglich, dass der Laser mit Hilfe von zumindest einem Spiegel ausgerichtet wird. Die korrigierten Offsetkoordinaten werden dann direkt als Ansteuersignale für den oder die Spiegel verstellende Motoren verwendet, um eine den Offset ausgleichende Grundstellung der Spiegel zu definieren. Grund hierfür ist, dass ein Motor genau eine x-Achsen-Korrektur macht und der andere Motor genau eine y-Achsen-Korrektur, und zwar an ein und demselben Spiegel oder an zwei Spiegeln, für jede Koordinate einen.

Bei dem erFndungsgemäßen System zum Ablatieren von Hornhaut an einem Auge umfasst die Zieleinrichtung eine Recheneinrichtung, die dazu ausgelegt ist, die zwei von dem Eyetracker erfassten Koordinaten zu korrigieren.

Bei der Erfindung ist die Recheneinheit dazu ausgelegt, die vor Abgabe eines Laserpulses von dem Eyetracker erfassten Koordinaten um einen ersten Faktor $k_1$ zu korrigieren und vor Abgabe weiterer Laserschüsse die Koordinaten unter Verwendung der Differenz zwischen den dann und den zunächst erfassten Koordinaten, multipliziert mit konstantem Faktor $k_2$, zu korrigieren.

Die Zieleinrichtung kann zumindest einen mit Motoren drehbaren Spiegel umfassen, wobei die Motoren jeweils durch Zuführen eines eine Koordinate wiedergebenden Signals ansteuerbar sind. Im Rahmen der Erfindung erhalten diese dann von der Recheneinrichtung Signale, welche die korrigierten Koordinaten wiedergeben (und nicht wie im Stand der Technik Signale, welche unkorrigierte Koordinaten wiedergeben).

Die Erfindung wird nachfolgend unter Bezug auf die Zeichnung näher beschrieben, wobei

FIG 1    schematisch den Aufbau eines Systems zum Ablatieren von Hornhaut an einem Auge veranschaulicht,

FIG 2    den Strahlengang anhand eines Ausschnitts aus FIG 1 veranschaulicht,

FIG 3    ein unverkipptes Auge darstellt, an dem mehrere Größen definiert werden,

FIG 4    ein verkipptes Auge darstellt, wobei in der Darstellung von FIG 4 die Größen aus FIG 3 zusätzlich wiedergegeben sind und weitere Größen definiert sind.

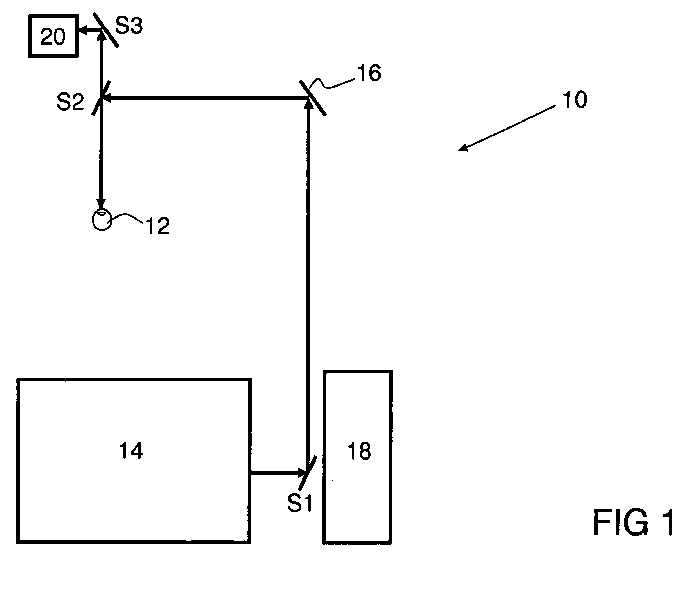

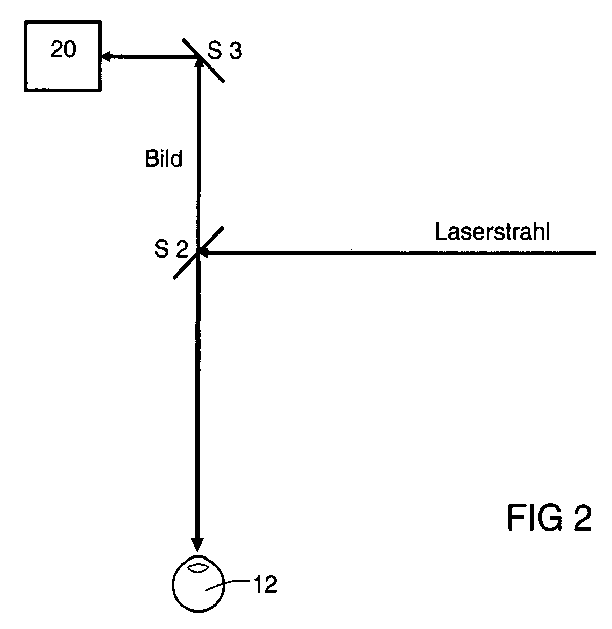

Ein in FIG 1 gezeigtes, im Ganzen mit 10 bezeichnetes System dient zum Ablatieren von Hornhaut an einem Auge 12. Auf die Hornhaut wird hierbei eine Folge von Laserpulsen von einem Laser 14 geschickt, wobei ein Spiegelsystem mit festen Spiegeln S1 und S2 und einer Scannereinheit 16 dafür sorgt, dass die einzelnen Laserpulse auf wohlbestimmte Stellen an dem Auge auftreffen. Um den Laserpuls auf verschiedene Stellen an dem Auge 12 senden zu können, und auch um verschiedene relative Lagen des Auges 12 zu berücksichtigen, erlaubt die Scannereinheit 16 als Teil einer Zieleinrichtung ein Nachfahren des Lasers längs einer x-Achse und einer y-Achse. In der Darstellung ist nur ein einziger Spiegel gezeigt, der die Scannereinheit 16 repräsentiert. Ein solcher Spiegel kann in zwei Achsen verkippbar sein. Alternativ ist es möglich, zwei Spiegel vorzusehen, wobei ein Spiegel eine Ablenkung zur Definition einer x-Koordinate und ein zweiter Spiegel eine Ablenkung des Laserstrahls zur Definition einer y-Koordinate, üblicherweise bezogen auf ein Koordinatsystem des Auges 12, erlaubt. Bei einem Verfahren der Scannereinheit 16 wird somit die Hornhaut des Auges 12 überstrichen. Eine Steuereinheit 18, die als Computer ausgeführt sein kann, steuert die Scannereinheit 16 an. Die Scannereinheit 16 kann auch eine Bewegung des Auges 12 ausgleichen, um zu gewährleisten, dass auf einen ganz bestimmten Punkt des Auges gezielt wird. Zur Überwachung der Augenbewegung dient ein Eyetracker 20. Wie FIG 2 genauer zu entnehmen, wird ein Bild des Auges 12 über den halbdurchlässigen Spiegel S2 und einen weiteren Spiegel S3 zu dem Eyetracker geleitet. Der Eyetracker leitet zwei Koordinaten ab, in x- und in y-Richtung, und zwar üblicherweise die Koordinaten des Zentrums der Pupille. Grund hierfür ist, dass in dem von dem Eyetracker 20 erfassten Bild die Pupille besonders gut zu erkennen ist. Der Scannereinheit 16 wird, ggf. auf einem Umweg über die Steuereinheit 18, die Ausgabe des Eyetrackers zugeführt, damit die Scannereinheit 16 die Augenbewegung ausgleicht.

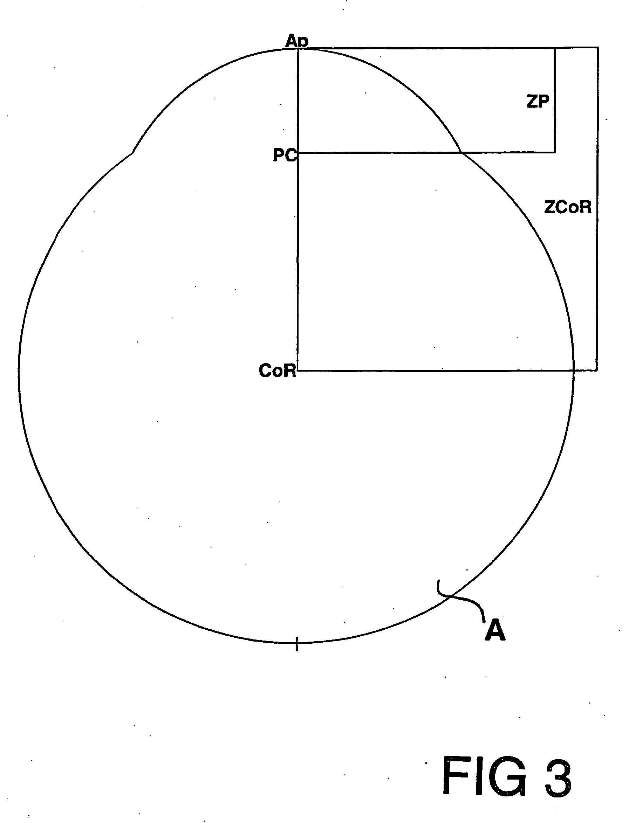

Die Lage des Zentrums der Pupille, PC, ist anhand eines Querschnitts eines Auges A in der FIG 3 dargestellt. Behandelt wird jedoch nicht die Pupille, sondern die Hornhaut. Ein ausgezeichneter Punkt auf der Hornhaut ist der so genannte Apex Ap. Der Apex ist die Stelle mit der größten Krümmung der Hornhaut und fällt typischerweise, so auch hier, mit der Stelle der höchsten Erhebung der Hornhaut zusammen, bezogen auf die Abweichung von der kreisförmigen Kontur, welche auch als "corneal vertex" bezeichnet wird. Der "corneal vertex" ist die Stelle auf der Hornhaut, die den größten Abstand zum Zentrum des Glaskörpers des Auges A hat, das in FIG 3 mit CoR bezeichnet ist, weil es gleichzeitig den Drehmittelpunkt des Auges bildet ("Center of Rotation"). Der Abstand zwischen CoR und dem Apex Ap sei mit ZCoR und der Abstand zwischen dem Zentrum der Pupille PC und dem Apex Ap sei mit ZP bezeichnet.

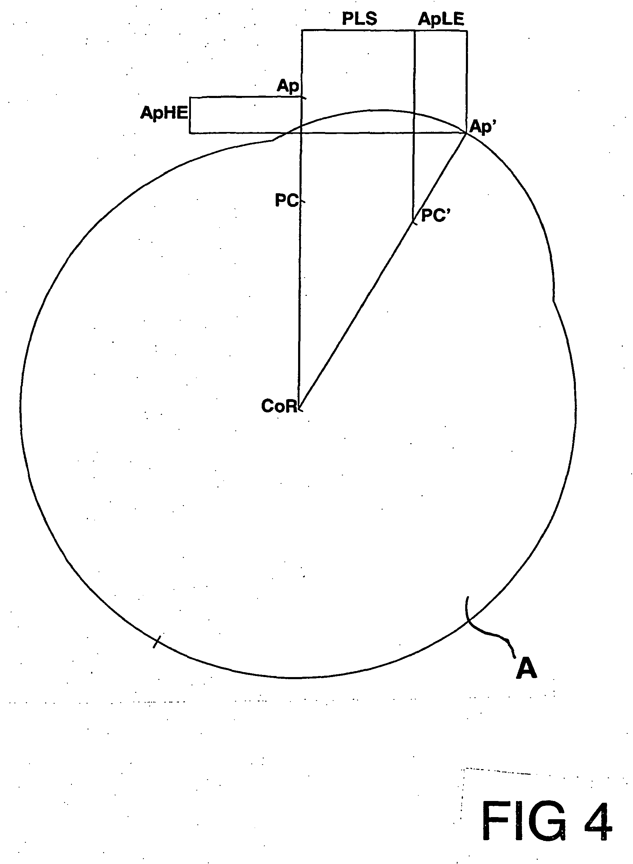

Wird das Auge A aus FIG 3 durch Drehung verkippt, ergibt sich die in FIG 4 gezeigte Situation. Der Eyetracker erfasst das Zentrum der Pupille und ermittelt somit die Koordinaten des Punktes PC'. Im Vergleich zu der Situation aus FIG 3 ermittelt er somit eine Verschiebung um die Strecke PLS (PLS steht für "pupil lateral shift", laterale Pupillenverschiebung). Bisher wird diese Verschiebung PLS direkt verwendet, um eine Offsetkoordinate zu definieren. Die Zieleinrichtung, die den Laserstrahl auf das Auge A leitet, wird somit derart verschoben, dass der Laserstrahl um die Offsetkoordinate PLS verschoben auf dem Auge auftrifft. Der Punkt, den der Laserstrahl treffen soll, sei insbesondere der Apex oder ein nah an diesem gelegener Punkt. FIG 4 ist somit klar zu entnehmen, dass der Laserstrahl, der in Richtung von PC' zielt, weit an dem tatsächlichen Punkt des "corneal vertex", also vorliegend des Apex, Ap', vorbeizielt. Anstelle einer Offsetkorrektur um den Wert PLS müsste eine Offsetkorrektur um den Wert PLS + ApLE vorgenommen werden. (Eine Korrektur in der Höhe um den Wert ApHE ist nicht unbedingt erforderlich.) Mit den Definitionen aus FIG 3 lässt sich nun ein Faktor ermitteln, um den PLS zu ändern ist, damit man den erforderlichen Wert PLS + ApLE erhält. Aus dem Strahlenersatz ergibt nämlich unmittelbar, dass sich PLS zu (PLS + ApLE) so verhält wie (ZCoR - ZP) zu zCoR PLS ist somit um einen Faktor k zu erhöhen, um den richtigen Offsetvektor zu erhalten, wobei $k = \dfrac{ZCoR}{ZCoR - ZP}$.

Dies gilt sowohl in der x-Achse (z. B. wie in FIG 3 und 4 gezeigt), als auch in der y-Achse, für die analoge Betrachtungen gelten.

Bei einem typischen Auge erhält man einen Wert von 3,75 mm für ZP und 13 mm für ZCoR. Somit ergibt sich: k = 1,4.

Je nach den Umständen, unter denen die Laserablation erfolgt, kann zusätzlich zu der anhand von FIG 3 und FIG 4 erläuterten Verkippung des Auges A auch eine seitliche Bewegung des Kopfes stattfinden, welche ebenfalls zu einem Offset führt, das nicht wie vorliegend zu korrigieren ist. Möchte man beide Einflüsse auf den Offset, die Verkippung des Auges und die Verschiebung des Kopfes, gleichgewichten, muss man den Faktor k somit erniedrigen, z. B. auf k = 1,2. Vor dem ersten Schuss ist die Hauptursache für den Offset nicht auf eine Augenverkippung zurückzuführen, so dass der Wert weiter reduziert werden sollte auf $k_1 = 1,1$.

Man erhält somit für den ersten Schuss die korrigierten Offsetkoordinaten:

$$x_{FS, korr} = \quad k_1 \cdot x_{FS, ET} = 1,1 \cdot x_{FS, ET}$$

$$y_{FS, korr} = k_1 \cdot y_{FS, ET} = 1{,}1 \cdot y_{FS, ET}$$

Das Kürzel "FS" steht für "First Shot", erster Laserschuss, und "ET" steht für "Eyetracker", um anzuzeigen, dass dies direkt von dem Eyetracker ermittelten Koordinaten des Zentrums der Pupille sind.

Die korrigierten Offsetkoordinaten für spätere Schüsse ("LS", Later Shot) ergeben sich aus den Offsetkoordinaten für den ersten Schuss und einer Gewichtung der zusätzlichen Abweichung mit einem größeren Faktor von $k_2$ gleich 1,2. Man erhält so:

$$
\begin{aligned}
x_{LS, korr} &= x_{FS, korr} + k_2 (x_{LS, ET} - x_{FS, ET}) \\
&= k_1 \cdot x_{FS, ET} + k_2 (x_{LS, ET} - x_{FS, ET}) \\
&= k_2 \cdot x_{LS, ET} - (k_2 - k_1) \cdot x_{FS, ET} \\
&= 1{,}2 \cdot x_{LS, ET} - 0{,}1 \cdot x_{FS, ET}
\end{aligned}
$$

$$
\begin{aligned}
y_{LS, korr} &= k_2 \cdot y_{LS, ET} - (k_2 - k_1) \cdot y_{FS, ET} \\
&= 1{,}2 \cdot y_{LS, ET} - 0{,}1 \cdot y_{FS, ET}
\end{aligned}
$$

Zusammenfassend lässt sich feststellen, dass anhand des Strahlensatzes der Einfluss der Verkippung genau berechenbar ist. Nicht genau ermittelbar ist der Anteil, den die Verkippung gegenüber einer einfachen Verschiebung durch Kopfbewegung hat. Die obigen Formeln stellen einen möglichen Ansatz dar, eine solche Gewichtung zu berücksichtigen, wobei zwischen dem ersten Schuss und weiteren Schüssen unterschieden wird. Für $k_1$ und $k_2$ können auch andere Werte gewählt werden.

**Patentansprüche**

1. System (10) zum Ablatieren von Hornhaut an einem Auge (12) mit:

   - einem Laser (14), der Laserpulse abgibt,
   - einer Zieleinrichtung (16) zum zielgenauen Lenken der Laserpulse auf die Augenhornhaut,
   - einem Eyetracker (20), der zwei Koordinaten der Position des Zentrums der Pupille des Auges erfasst und der Zieleinrichtung Eingabedaten zum Herstellen einer Grundstellung zuführt,

   wobei die Zieleinrichtung eine Recheneinrichtung (18) umfasst, die dazu ausgelegt ist, die zwei von dem Eyetracker (20) erfassten Koordinaten zu korrigieren,
   **dadurch gekennzeichnet,**
   **dass** die Recheneinheit dazu ausgelegt ist, die vor der Abgabe eines ersten Laserpulses von dem Eyetracker (20) erfassten Koordinaten $x_{FS, ET}$ und $y_{FS, ET}$ um einen ersten konstanten Faktor $k_1$ mit $1{,}0 \le k_1 \le 1{,}2$ zu korrigieren zu $x_{FS, korr} = k_1 x_{FS, ET}$, $y_{FS, korr} = k_1 y_{FS, ET}$ und vor Abgabe weiterer Laserschüsse die dann von dem Eyetracker (20) erfassten Koordinaten $x_{LS, ET}$, $y_{LS, ET}$ zu korrigieren gemäß

$$
\begin{aligned}
x_{LS, korr} &= x_{FS, korr} + k_2 (x_{LS, ET} - x_{FS, ET}) \\
&= k_1 \cdot x_{FS, ET} + k_2 (x_{LS, ET} - x_{FS, ET}) \\
&= k_2 \cdot x_{LS, ET} - (k_2 - k_1) \cdot x_{FS, ET}
\end{aligned}
$$

$$
y_{LS, korr} = k_2 \cdot y_{LS, ET} - (k_2 - k_1) \cdot y_{FS, ET}
$$

mit konstantem Faktor $k_2$ , wobei $1{,}2 \leq k_2 \leq 1{,}5$ und $k_2 \geq k_1$. derart, dass die Zieleinrichtung (16) in der Grundstellung genau einen Offset ausgleicht, der durch eine Bewegung des Auges entstanden ist und durch die korrigierten Koordinaten quantifiziert ist.

2. System (10) nach Anspruch 1,
**dadurch gekennzeichnet,**
**dass** die Zieleinrichtung zumindest einen mit Motoren beweglichen Spiegel umfasst, wobei die Motoren jeweils durch Zuführen eines eine Koordinate wiedergebenden Signals ansteuerbar sind und von der Recheneinrichtung (18) Signale erhalten, welche die korrigierten Koordinaten wiedergeben.

**Claims**

1. System (10) for ablating cornea on an eye (12) including:

   - a laser (14) emitting laser pulses,
   - a targeting device (16) for precisely targeted directing the laser pulses onto the eye's cornea,
   - an eye tracker (20) acquiring two coordinates of the position of the center of the pupil of the eye and supplying input data for establishing a basic position to the targeting device,

   wherein the targeting device includes a computing device (18) configured to correct the two coordinates acquired by the eye tracker (20), **characterized in that**
   the computing device is configured to correct the coordinates $X_{FS,ET}$ and $y_{FS,ET}$ acquired by the eye tracker (20) before emission of a first laser pulse by a first constant factor $k_1$ with $1.0 \leq k_1 \leq 1.2$ to $X_{FS,korr} = k_1 X_{FS,ET}$, $Y_{FS,korr} = k_1 Y_{FS,ET}$ and to correct the coordinates $X_{LS,ET}$ and $Y_{LS,ET}$ then acquired by the eye tracker (20) before emission of further laser shots according to

$$x_{LS,\,korr} = x_{FS,\,korr} + k_2 \left( x_{LS,\,ET} - x_{FS,\,ET} \right)$$
$$= k_1 \cdot x_{FS,\,ET} + k_2 \left( x_{LS,\,ET} - x_{FS,\,ET} \right)$$
$$= k_2 \cdot x_{LS,\,ET} - (k_2 - k_1) \cdot x_{FS,\,ET}$$

$$y_{LS,\,korr} = k_2 \cdot y_{LS,\,ET} - (k_2 - k_1) \cdot y_{FS,\,ET}$$

with constant factor $k_2$, wherein $1.2 \leq k_2 \leq 1.5$ and $k_2 \geq k_1$ such that the targeting device (16) exactly compensates for an offset in the basic position, which has arisen by movement of the eye and is quantified by the corrected coordinates.

2. System (10) according to claim 1,
**characterized in that**
the targeting device includes at least one mirror movable by motors, wherein the motors are each drivable by supplying a signal reproducing a coordinate and obtain signals from the computing device (18), which reproduce the corrected coordinates.

**Revendications**

1. Système (10) destiné à l'ablation de la cornée d'un oeil (12), avec :

   - un laser (14), qui délivre des impulsions laser,
   - un dispositif de visée (16), pour l'orientation précise des impulsions laser sur la cornée de l'oeil,
   - un oculomètre (20), qui saisit deux coordonnées de la position du centre de la pupille de l'oeil et amène au dispositif de visée des données d'entrée pour constituer une position de base,

moyennant quoi le dispositif de visée comprend un dispositif de calcul (18), qui est conçu pour corriger les deux coordonnées, saisies par l'oculomètre (20),

**caractérisé en ce**

**que** l'unité de calcul est conçue pour corriger les coordonnées $x_{FS, ET}$ et $y_{FS}$, ET, saisies avant la délivrance d'une première impulsion laser par l'oculomètre (20), d'un premier facteur $k_1$ constant avec $1,0 \leq k_1 \leq 1,2$ par rapport à $x_{FS, korr} = k_{1 \cdot xFS, ET}, y_{FS, korr} = k_1 \cdot y_{FS, ET}$ et pour corriger, avant la délivrance d'autres tirs lasers, les coordonnées $x_{LS, ET}, y_{LS, ET}$, saisies alors par l'oculomètre (20), conformément à

$$x_{LS, korr} \quad = \quad x_{FS, korr} + k_2 (x_{LS, ET} - x_{FS, ET})$$

$$= \quad k_1 \cdot x_{FS, ET} + k_2 (x_{LS, ET} - x_{FS, ET})$$

$$= \quad k_2 \cdot x_{LS, ET} - (k_2 - k_1) \cdot x_{FS, ET}$$

$$y_{LS, korr} \quad = \quad k_2 \cdot y_{LS, ET} - (k_2 - k_1) \cdot y_{FS, ET},$$

avec le facteur $k_2$ constant, moyennant quoi $1,2 \leq k_2 \leq 1,5$ et $k_2 \geq k_1$, de telle sorte que le dispositif de visée (16) compense exactement, dans la position de base, un décalage qui est apparu par le biais d'un mouvement de l'oeil et qui est quantifié par le biais des coordonnées corrigées.

2. Système (10) selon la revendication 1,
**caractérisé en ce**
**que** le dispositif de visée comprend au moins un miroir, mobile avec des moteurs, moyennant quoi les moteurs peuvent être activés respectivement par l'apport d'un signal, qui retransmet une coordonnée et reçoivent du dispositif de calcul (18) des signaux, lesquels retransmettent les coordonnées corrigées.

FIG 1

EP 1 923 027 B1

FIG 2

EP 1 923 027 B1

Ap

ZP

PC

ZCoR

CoR

A

# FIG 3

FIG 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2005099639 A1

- WO 2005099639 A